# EUROPEAN PATENT APPLICATION

(11) **EP 4 414 387 A1**
(43) Date of publication of application: **14.08.2024**
(21) Application number: 22877933.6
(22) Date of filing: 07.10.2022
(51) Int. Cl.: C07K 16/28, C07K 16/00, A61K 39/00, A61K 39/395, C12P 21/08, A61P 35/00

(54) **ANTI-FGFR2 ADCC ENHANCED ANTIBODY AND USE THEREOF**

(30) Priority: 08.10.2021 WO PCT/CN2021/122699
(71) Applicant: Shenzhen Forward Pharmaceuticals Co., Ltd., Shenzhen, Guangdong 518067 (CN)
(72) Inventor: ZHU, Chenggang, Shenzhen, Guangdong 518057 (CN); DONG, Jiexian, Shenzhen, Guangdong 518057 (CN); PENG, Fangli, Shenzhen, Guangdong 518057 (CN); LI, Yeqing, Shenzhen, Guangdong 518057 (CN); YANG, Chaoming, Shenzhen, Guangdong 518057 (CN); XU, Liangliang, Shenzhen, Guangdong 518057 (CN)
(74) Representative: Zwicker, Jörk
(86) International application number: PCT/CN2022/123723
(87) International publication number: WO 2023/056916

(57) **Abstract**

Provided in the present invention is an anti-FGFR2 ADCC enhanced antibody. The antibody achieves ADCC enhancement by means of enhancing the binding to FcgammaR. In tumor therapy, the antibody has anti-tumor activity.

## Description

### Technical Field

The present invention relates to an ADCC-enhanced anti-FGFR2 antibody and use thereof.

### Background Art

In normal cells, fibroblast growth factor (FGF) receptor 2 (FGFR2) can transduce FGF signals to the RAS-ERK and PI3K-AKT signaling pathways through the FRS2 cascade, and also regulate cell differentiation, proliferation, and apoptosis by activating the DAG-PKC and IP3-calmodulin signaling cascades with PLCγ.

The coding gene for FGFR2 is distributed on human chromosome 10q26, and due to splicing changes, FGFR2b and FGFR2c isoforms are produced. The immunoglobulin-like domains and intracellular tyrosine kinase domains of FGFR2b and FGFR2c are almost identical, with the main difference being in the latter half of the third immunoglobulin-like domain. In addition, the main expressing cells and binding ligands of the two isoforms are also different, wherein FGFR2b, which is mainly expressed in epithelial cells, binds strongly to ligands FGF1, FGF3, FGF7, FGF10, and FGF22, and FGFR2c, which is expressed in mesenchymal cells, mainly binds to ligands FGF1, FGF2, FGF4, FGF6, FGF9, FGF16, and FGF20.

Overexpression of FGFR2 or FGFs, as well as genetic changes such as gene amplification, gene fusion and rearrangement, gene point mutations, and chromosomal translocation, can lead to dysregulation of the FGFR2 signaling pathway, and the dysregulated FGFRs/FGFs signaling pathway is closely related to cellular carcinogenesis. Potential overexpression, activation by missense mutation, or abnormal protein fusion of FGFR2 have been reported in a variety of cancer types, including endometrial cancer, ovarian cancer, breast cancer, lung cancer, gastric cancer, and bile duct cancer. Therefore, FGFR2 can become a potential target for tumor treatment.

Currently, highly specific anti-tumor drugs targeting FGFR2 are undergoing preclinical and clinical trials. These drugs are classified according to their mechanisms of action as receptor tyrosine kinase inhibitors, antagonistic antibodies, or peptide inhibitors. Among them, several monoclonal antibodies targeting FGFR2 are in the preclinical or clinical development stage. For example, the FGFR2IIIb-specific antibody GP369 has been shown to inhibit the proliferation of human cancer cell lines and tumor xenografts by amplified or activated FGFR2 signals. BAY1187982 (Bayer) had been shown to be effective in preclinical research by adopting the therapeutic strategy of antibody-drug conjugates. It successfully inhibited tumor growth in the xenotransplantation models of gastric cancer and breast cancer with overexpression of FGFR2. Unfortunately, the Phase 1 clinical trial of the drug was terminated in 2016 due to safety concerns. The antibody FP144 (Five Prime) targeting FGFR2IIIb has been shown to inhibit FGF/FGFR2-related tumor signaling pathways, thereby inhibiting tumor growth. Phase II clinical trial data shows that in gastric cancer with overexpression of FGFR2, the combination therapy of FP144 and chemotherapeutic drugs demonstrates positive therapeutic effects.

To improve the therapeutic effects of antibody drugs, many pharmaceutical companies and research institutions have made various attempts. Among them, antibody Fc modification has been proven to be an effective approach to enhance antibody-dependent cell-mediated cytotoxicity (ADCC), thereby enhancing anti-tumor activity. Fucose knockout at Asn297 or Fc point mutations are mainly used in Fc modification for enhancing ADCC.

IgG1 subtype antibodies are widely used in cancer immunotherapy, and there is a conserved N-linked glycosylation site at Asn297 of the CH2 domain of IgG1 subtype antibodies. Studies have shown that removing the N-linked glycan at this site can enhance binding of the antibody Fc end to FcyRs on NK cells, thereby enhancing ADCC activity. In 1999, Umana, P. *et al.* reported that the glycosylation content at Asn297 of the antibody produced using a cell line, in which the β(1,4)-N-acetylglucosaminyltransferase III was knocked out in Chinese hamster ovarian cells, decreased, and the antibody exhibited significantly enhanced ADCC activity *in vitro.* So far, multiple afucosylated antibody drugs have been approved for marketing or are in the clinical stage. For example, Obinutuzumab targeting CD20 developed by Roche and Mogamulizumab targeting CCR4 developed by Kyowa Hakko Kirin have been approved for marketing in the United States and Europe.

In addition to knocking out fucose, another proven effective strategy is to introduce a point mutation at the Fc end, in order to develop mutants with high affinity for FcyRs. Based on *in silicon* interaction analysis, Xencor found that when S239D/I332E or S239D/I332E/A330L were introduced into IgG1 Fc, the results showed that compared to the wild-type, the mutant exhibited an affinity for FcyRs increased by 100 times, and significantly enhanced ADCC activity.

### Summary of the Invention

The present invention provides an ADCC-enhanced anti-FGFR2 antibody and use thereof. In one embodiment, ADCC is enhanced by enhancing binding to FcyRs.

In one aspect, the present invention provides an ADCC-enhanced anti-FGFR2 antibody, wherein compared to a wild-type Fc region that is fucosylated, the Fc region of the antibody exhibits enhanced binding to FcyRs.

In one embodiment, the heavy chain variable region of the antibody comprises an HVR-H1 as set forth in SEQ ID NO: 5, an HVR-H2 as set forth in SEQ ID NO: 6, and an HVR-H3 as set forth in SEQ ID NO: 7. In one embodiment, the light chain variable region of the antibody comprises an HVR-L1 as set forth in SEQ ID NO: 8, an HVR-L2 as set forth in SEQ ID NO: 9, and an HVR-L3 as set forth in SEQ ID NO: 10. In one embodiment, the antibody comprises a heavy chain variable region as set forth in SEQ ID NO: 3. In one embodiment, the antibody comprises a light chain variable region as set forth in SEQ ID NO: 4. In one embodiment, the antibody comprises a heavy chain variable region as set forth in SEQ ID NO: 3, and a light chain variable region as set forth in SEQ ID NO: 4. In one embodiment, the antibody comprises a κ light chain constant region. In one embodiment, the antibody comprises an IgG1 heavy chain constant region.

In one embodiment, the Fc region of the antibody comprises an amino acid mutation that eliminates fucosylation, such as N297G or N297A. In one embodiment, the Fc region of the antibody comprises an amino acid mutation that enhances binding to FcyRs, such as S239D/I332E or S239D/I332E/A330L. In one embodiment, the antibody comprises a heavy chain as set forth in SEQ ID NO: 12 or 14. In one embodiment, the antibody comprises a light chain as set forth in SEQ ID NO: 11. In one embodiment, the antibody comprises a heavy chain as set forth in SEQ ID NO: 12, and a light chain as set forth in SEQ ID NO: 11. In one embodiment, the antibody comprises a heavy chain as set forth in SEQ ID NO: 14, and a light chain as set forth in SEQ ID NO: 11.

In one embodiment, the antibody is generated in a host cell deficient in protein fucosylation. In one embodiment, the host cell is α-1,6-fucosyltransferase (FUT8)-deficient, such as gene knockout, decreased expression, and/or decreased activity. In one embodiment, the antibody is generated under conditions where protein fucosylation is inhibited. In one embodiment, the conditions inhibit the expression and/or activity of α-1,6-fucosyltransferase (FUT8). In one embodiment, the host cell is a prokaryotic cell or a eukaryotic cell. In one embodiment, the host cell is *Escherichia coli.* In one embodiment, the cell is a mammalian cell. In one embodiment, the host cell is a CHO cell, such as a CHOK1 cell. In one embodiment, the conditions comprise the addition of an inhibitor of α-1,6-fucosyltransferase (FUT8).

In another aspect, the present invention provides a composition, comprising the ADCC-enhanced anti-FGFR2 antibody of the present invention. In one embodiment, at least 50%, 60%, 70%, 75%, 80%, 85%, 90%, 91%, 92%, 93%, 94%, 95%, 96%, 97%, 98%, 99%, or even 100% of the anti-FGFR2 antibodies in the composition are afucosylated.

In another aspect, the present invention provides a nucleic acid encoding the ADCC-enhanced anti-FGFR2 antibody of the present invention. In another aspect, the present invention provides a vector, comprising the nucleic acid of the present invention. In one embodiment, the vector is a cloning vector or an expression vector. In another aspect, the present invention provides a host cell, comprising the nucleic acid or vector of the present invention.

In another aspect, the present invention provides a method for generating the ADCC-enhanced anti-FGFR2 antibody of the present invention or the composition comprising the ADCC-enhanced anti-FGFR2 antibody of the present invention. In one embodiment, the method comprises generating the antibody of the present invention in a host cell deficient in protein fucosylation, in order to produce the ADCC-enhanced anti-FGFR2 antibody or composition of the present invention. In one embodiment, the method comprises culturing the host cell of the present invention under conditions where protein fucosylation is inhibited, in order to produce the ADCC-enhanced anti-FGFR2 antibody or composition of the present invention. In one embodiment, the method comprises culturing the host cell of the present invention under conditions suitable for antibody production, in order to produce the ADCC-enhanced anti-FGFR2 antibody or composition of the present invention, wherein the host cell comprises a nucleic acid encoding an antibody with the fucosylation site deleted. In one embodiment, the host cell is a prokaryotic cell or a eukaryotic cell. In one embodiment, the host cell is *Escherichia coli.* In one embodiment, the cell is a mammalian cell. In one embodiment, the host cell is a CHO cell, such as a CHOK1 cell. In one embodiment, the host cell deficient in protein fucosylation is α-1,6-fucosyltransferase (FUT8)-deficient. In one embodiment, the deficiency of α-1,6-fucosyltransferase (FUT8) is achieved through gene knockout or RNA interference. In one embodiment, the conditions under which protein fucosylation is inhibited are the inhibition of the expression and/or activity of α-1,6-fucosyltransferase (FUT8). In one embodiment, the inhibition of the expression and/or activity of α-1,6-fucosyltransferase (FUT8) is achieved through the addition of an inhibitor of α-1,6-fucosyltransferase (FUT8).

In another aspect, the present invention provides a pharmaceutical composition, comprising the ADCC-enhanced anti-FGFR2 antibody or composition thereof of the present invention, and at least one pharmaceutically acceptable carrier.

In another aspect, the present invention provides the ADCC-enhanced anti-FGFR2 antibody, composition, or pharmaceutical composition of the present invention, for use in treating cancer. In another aspect, the present invention provides use of the ADCC-enhanced anti-FGFR2 antibody, composition, or pharmaceutical composition of the present invention for treating cancer. In another aspect, the present invention provides use of the ADCC-enhanced anti-FGFR2 antibody, composition, or pharmaceutical composition of the present invention in the preparation of a medicament for treating cancer. In another aspect, the present invention provides a method for treating cancer, wherein the method comprises administering an effective amount of the ADCC-enhanced anti-FGFR2 antibody, composition, or pharmaceutical composition of the present invention. In one embodiment, the cancer overexpresses FGFR2. In one embodiment, the cancer is gastric cancer or breast cancer. In one embodiment, the medicament is administered in combination with other therapeutic agents.

### Brief Description of the Drawings

Figure 1 shows the antigen binding activity of the antibody of the present invention.
Figure 2 shows the ADCC activity of the antibody of the present invention, as detected by using Jurkat as the effector cell. Panel A, KATOIII used as the target cell; Panel B, SUM52PE used as the target cell; and Panel C, SNU16 used as the target cell.
Figure 3 shows the ADCC activity of the antibody of the present invention, as detected by using human PBMC as the effector cell. Panel A, KATOIII used as the target cell; Panel B, SUM52PE used as the target cell; and Panel C, SNU16 used as the target cell.

### Detailed Description of Embodiments

The present invention provides an afucosylated antibody that binds to FGFR2IIIb. In some embodiments, afucosylated antibody heavy and light chains capable of forming an antibody that binds to FGFR2IIIb are also provided. In some embodiments, an afucosylated antibody, heavy chain, and light chain comprising one or more specific hypervariable regions (HVRs) are provided. In some embodiments, compared to a fucosylated anti-FGFR2IIIb antibody, the afucosylated anti-FGFR2IIIb antibody exhibits enhanced ADCC activity. In some embodiments, compared to a fucosylated anti-FGFR2IIIb antibody, the anti-FGFR2IIIb antibody with S239D, I332E, and A330L mutations in the antibody Fc region exhibits enhanced ADCC activity. In some embodiments, compared to a fucosylated anti-FGFR2IIIb antibody, the afucosylated anti-FGFR2IIIb antibody with S239D, I332E, and A330L mutations exhibits enhanced ADCC activity.

A polynucleotide encoding the antibody that binds to FGFR2IIIb is provided. A polynucleotide encoding the antibody heavy or light chain is also provided. A host cell expressing the afucosylated anti-FGFR2IIIb antibody is provided. A method of treatment using the afucosylated anti-FGFR2IIIb antibody with S239D, I332E, and A330L mutations is provided. Such a method includes, but is not limited to, methods for treating cancers, such as gastric cancer, breast cancer, ovarian cancer, endometrial cancer, pancreatic cancer, and esophageal cancer.

The present invention provides an antibody targeting FGFR2.

The present invention also provides a drug combination comprising the antibody of the present invention, and use of the antibody in the preparation of a medicament for diagnosing or treating gastric cancer, especially gastric cancer caused by overexpression of FGFR2IIIb due to gene amplification of FGFR2IIIb.

Specifically, the present invention encompasses an antibody that binds to FGFR2IIIb and is oligoglycosylated at Asn297, characterized by a fucose content of 75% or more in the oligosaccharide chain. The present invention also provides an ADCC-enhanced anti-FGFR2 antibody, including one in which the fucose at Asn297 of the antibody is knocked out; or S239D/I332E/A330L mutations are introduced at the Fc end of the antibody; or S239D/I332E/A330L mutations are introduced at the Fc end of the afucosylated antibody, which set of antibodies is characterized by enhanced ADCC activity.

In any of the embodiments described in this application, the antibody may bind to FGFR2IIIb rather than FGFR2IIIc.

In some embodiments, the antibody is a monoclonal antibody. In some embodiments, the antibody is a chimeric antibody. In some embodiments, the antibody is a humanized antibody. In any of the embodiments described in this application, the antibody may comprise a κ light chain constant region. In any of the embodiments described in this application, the antibody may comprise an IgG1 heavy chain constant region.

In some embodiments, an anti-FGFR2IIIb antibody is provided, wherein the heavy chain variable region comprises: (i) an HVR-H1 comprising the amino acid sequence of SEQ ID NO: 5; (ii) an HVR-H2 comprising the amino acid sequence of SEQ ID NO: 6; and (iii) an HVR-H3 comprising the amino acid sequence of SEQ ID NO: 7; and the light chain variable region comprises: (iv) an HVR-L1 comprising the amino acid sequence of SEQ ID NO: 8; (v) an HVR-L2 comprising the amino acid sequence of SEQ ID NO: 9; and (vi) an HVR-L3 comprising the amino acid sequence of SEQ ID NO: 10. In some embodiments, the antibody lacks fucose at Asn297. In some embodiments, S239D/I332E/A330L mutations are introduced at the Fc end of the antibody, whose Fc sequence comprises SEQ ID NO: 13. In some embodiments, a composition comprising a plurality of anti-FGFR2IIIb antibodies is provided, wherein the heavy chain CDR region of each anti-FGFR2IIIb antibody in the composition comprises: (i) an HVR-H1 comprising the amino acid sequence of SEQ ID NO: 5; (ii) an HVR-H2 comprising the amino acid sequence of SEQ ID NO: 6; and (iii) an HVR-H3 comprising the amino acid sequence of SEQ ID NO: 7; and the light chain CDR region of each anti-FGFR2IIIb antibody in the composition comprises: (iv) an HVR-L1 comprising the amino acid sequence of SEQ ID NO: 8; (v) an HVR-L2 comprising the amino acid sequence of SEQ ID NO: 9; and (vi) an HVR-L3 comprising the amino acid sequence of SEQ ID NO: 10; and wherein at least 95% of the antibodies in the composition are afucosylated, or S239D/I332E/A330L mutations are introduced at the Fc end of the antibodies in the composition. In some embodiments, the composition may be a supernatant from an antibody-producing cell line. In some embodiments, the composition may be a buffered composition. In some embodiments, the heavy chain variable domain comprises the amino acid sequence of SEQ ID NO: 3. In some embodiments, the light chain variable domain comprises the amino acid sequence of SEQ ID NO: 4. In some embodiments, the heavy chain variable domain comprises the amino acid sequence of SEQ ID NO: 3, and the light chain variable domain comprises the amino acid sequence of SEQ ID NO: 4. In some embodiments, the heavy chain comprises the amino acid sequence of SEQ ID NO: 12. In some embodiments, the light chain comprises the amino acid sequence of SEQ ID NO: 11. In some embodiments, the heavy chain comprises the amino acid sequence of SEQ ID NO: 12, and the light chain comprises the amino acid sequence of SEQ ID NO: 11. In some embodiments, the heavy chain comprises the amino acid sequence of SEQ ID NO: 14, and the light chain comprises the amino acid sequence of SEQ ID NO: 11. In some embodiments, a composition comprising a plurality of afucosylated anti-FGFR2IIIb antibodies is provided, wherein the antibodies compete with antibodies comprising the following heavy and light chain variable domains for binding to FGFR2IIIb: the heavy chain variable domain comprises the amino acid sequence of SEQ ID NO: 3, and the light chain variable domain comprises the amino acid sequence of SEQ ID NO: 4; or the heavy chain of the antibodies comprises the amino acid sequence of SEQ ID NO: 12, and the light chain of the antibodies comprises the amino acid sequence of SEQ ID NO: 11; or the heavy chain of the antibodies comprises the amino acid sequence of SEQ ID NO: 14, and the light chain of the antibodies comprises the amino acid sequence of SEQ ID NO: 11.

In some embodiments, the afucosylated antibody exhibits enhanced ADCC activity *in vitro* as compared to a fucosylated anti-FGFR2IIIb antibody with the same amino acid sequence. In some embodiments, the specific lysis caused by the afucosylated anti-FGFR2IIIb antibody is at least 10%, at least 15%, at least 20%, at least 25%, at least 30%, at least 35%, at least 40%, at least 45%, at least 50%, at least 60%, at least 65%, at least 70%, or at least 75% higher than that caused by a fucosylated anti-FGFR2IIIb antibody. In some embodiments, the ADCC activity is determined by using SNU16, KATOIII, and SUM52PE cells expressing FGFR2IIIb as target cells, and isolated human PBMCs as effector cells.

In some embodiments, the antibody exhibits enhanced ADCC activity *in vitro* as compared to an anti-FGFR2IIIb antibody with the same amino acid sequence and S239D/I332E/A330L mutations introduced at the Fc end. In some embodiments, the specific lysis caused by the anti-FGFR2IIIb antibody with S239D/I332E/A330L mutations introduced at the Fc end is at least 10%, at least 15%, at least 20%, at least 25%, at least 30%, at least 35%, at least 40%, at least 45%, at least 50%, at least 60%, at least 65%, at least 70%, or at least 75% higher than that caused by the original anti-FGFR2IIIb antibody. In some embodiments, the ADCC activity is determined by using SNU16, KATOIII, and SUM52PE cells expressing FGFR2IIIb as target cells, and isolated human PBMCs as effector cells.

In some embodiments, the afucosylated anti-FGFR2IIIb antibody with S239D/I332E/A330L mutations introduced at the Fc end exhibits enhanced ADCC activity *in vitro* as compared to an antibody with the same amino acid sequence. In some embodiments, the specific lysis caused by the afucosylated anti-FGFR2IIIb antibody with S239D/I332E/A330L mutations introduced at the Fc end is at least 10%, at least 15%, at least 20%, at least 25%, at least 30%, at least 35%, at least 40%, at least 45%, at least 50%, at least 60%, at least 65%, at least 70%, or at least 75% higher than that caused by the original anti-FGFR2IIIb antibody. In some embodiments, the ADCC activity is determined by using SNU16 (a gastric cancer cell line), KATOIII (a gastric cancer cell line), and SUM52PE (a breast cancer cell line) cells expressing FGFR2IIIb as target cells, and isolated human PBMCs as effector cells.

In some embodiments, a host cell is provided, comprising a nucleic acid encoding the anti-FGFR2IIIb antibody of this application, wherein the host cell lacks the functional α-1,6-fucosyltransferase (FUT8) gene. In some embodiments, the host cell is a CHO cell.

In some embodiments, a method for manufacturing the afucosylated anti-FGFR2IIIb antibody is provided. In some embodiments, the method comprises culturing the host cell under conditions suitable for expressing a gene encoding the anti-FGFR2IIIb antibody, wherein the host cell lacks the functional α-1,6-fucosyltransferase (FUT8) gene. In some embodiments, the method for manufacturing the afucosylated anti-FGFR2IIIb antibody comprises culturing the host cell under conditions suitable for producing the afucosylated anti-FGFR2IIIb antibody. In some embodiments, the method further comprises recovering the anti-FGFR2IIIb antibody produced by the host cell. In some embodiments, less than 5% of the anti-FGFR2IIIb antibodies produced by the host cell comprise fucose. In some embodiments, at least 95% of the anti-FGFR2IIIb antibodies produced by the host cell lack fucose *(i.e.,* afucosylated). In some embodiments, fucose cannot be detected in the anti-FGFR2IIIb antibodies produced by the host cell. In some embodiments, the presence of fucose is determined by mass spectrometry.

In some embodiments, a pharmaceutical composition is provided, wherein the pharmaceutical composition comprises the ADCC-enhanced anti-FGFR2IIIb antibody of this application, and a pharmaceutically acceptable carrier.

In some embodiments, a method for treating cancer is provided. In some embodiments, the method comprises administering an effective amount of the pharmaceutical composition comprising the ADCC-enhanced anti-FGFR2IIIb antibody of this application, and a pharmaceutically acceptable carrier. In some embodiments, the cancer is selected from gastric cancer, breast cancer, ovarian cancer, endometrial cancer, pancreatic cancer, and esophageal cancer. In some embodiments, the cancer is gastric cancer or breast cancer. In some embodiments, the cancer comprises amplified FGFR2 gene. In some embodiments, the cancer overexpresses FGFR2IIIb. In some embodiments, the cancer comprising overexpressed FGFR2 has a higher degree of overexpression of FGFR2IIIb than FGFR2IIIc. In some embodiments, the cancer overexpresses FGFR2IIIb, but does not comprise amplified FGFR2 gene. In some embodiments, the expression or overexpression of FGFR2IIIb is determined by IHC. In some embodiments, IHC staining of tumor cells as 1+, 2+, or 3+ indicates overexpression of FGFR2IIIb. In some embodiments, IHC staining of tumor cells as 2+ or 3+ indicates overexpression of FGFR2IIIb.

In some embodiments, the method for treating cancer further comprises administering at least one additional therapeutic agent selected from a platinum agent, paclitaxel, docetaxel, gemcitabine, capecitabine, irinotecan, epirubicin, FOLFOX, FOLFIRI, calcium folinate, fluorouracil, mitomycin C, and doxorubicin hydrochloride in combination. In some embodiments, the platinum agent is selected from cisplatin, oxaliplatin, and carboplatin. In some embodiments, the method for treating cancer further comprises administering paclitaxel. In some embodiments, the method for treating cancer further comprises administering cisplatin and/or 5-FU.

### Terminology

"FGFR2IIIb" or "FGFR2b" are used interchangeably to refer to the splicing form IIIb of fibroblast growth factor receptor 2. A non-limiting exemplary amino acid sequence of mature human FGFR2IIIb is as set forth in SEQ ID NO: 1.

"FGFR2IIIc" or "FGFR2c" are used interchangeably to refer to the splicing form IIIc of fibroblast growth factor receptor 2. A non-limiting exemplary amino acid sequence of mature human FGFR2IIIc is as set forth in SEQ ID NO: 2.

The term "antibody" herein is used in the broadest sense, and covers various antibody structures, including, but not limited to, monoclonal antibodies, polyclonal antibodies, multispecific antibodies (e.g., bispecific antibodies), and antibody fragments, so long as they exhibit the desired antigen binding activity.

The term "antibody" includes, but is not limited to, fragments that are capable of binding to an antigen, such as Fv, single-chain Fv (scFv), Fab, Fab', and (Fab')2. Papain digestion of antibodies produces two identical antigen-binding fragments, each with a single antigen-binding site, and a residual "Fc" fragment, whose name reflects its ability to crystallize readily. Pepsin treatment yields an F(ab')2 fragment that has two antigen-binding sites and is still capable of cross-linking an antigen. The term "antibody" also includes, but is not limited to, chimeric antibodies, humanized antibodies, and antibodies of various species such as mouse, human, macaque, *etc.*

The term "heavy chain variable region" refers to a region comprising heavy chain HVR1, framework (FR) 2, HVR2, FR3, and HVR3. In some embodiments, a heavy chain variable region also comprises at least a portion of an FR1 and/or at least a portion of an FR4.

The term "heavy chain constant region" refers to a region comprising at least three heavy chain constant domains, CH1, CH2, and CH3. Non-limiting exemplary heavy chain constant regions include γ, δ, and α. Non-limiting exemplary heavy chain constant regions also include ε and µ. Each heavy chain constant region corresponds to an antibody isotype. For example, an antibody comprising a γ constant region is an IgG antibody, an antibody comprising a δ constant region is an IgD antibody, and an antibody comprising an α constant region is an IgA antibody. Further, an antibody comprising a µ constant region is an IgM antibody, and an antibody comprising an ε constant region is an IgE antibody. Certain isotypes can be further subdivided into subclasses. For example, IgG antibodies include, but are not limited to, IgG1 (comprising a γ1 constant region), IgG2 (comprising a γ2 constant region), IgG3 (comprising a γ3 constant region), and IgG4 (comprising a γ4 constant region) antibodies; IgA antibodies include, but are not limited to, IgA1 (comprising an α1 constant region) and IgA2 (comprising an α2 constant region) antibodies; and IgM antibodies include, but are not limited to, IgM1 and IgM2.

The term "heavy chain" refers to a polypeptide comprising at least a heavy chain variable region, with or without a leader sequence. In some embodiments, a heavy chain comprises at least a portion of a heavy chain variable region. The term "full-length heavy chain" refers to a polypeptide comprising a heavy chain variable region and a heavy chain constant region, with or without a leader sequence.

The term "light chain variable region" refers to a region comprising light chain HVR1, framework (FR) 2, HVR2, FR3, and HVR3. In some embodiments, a light chain variable region also comprises an FR1 and/or an FR4.

The term "light chain constant region" refers to a region comprising a light chain constant domain, CL. Non-limiting exemplary light chain constant regions include λ and κ.

The term "light chain" refers to a polypeptide comprising at least a light chain variable region, with or without a leader sequence. In some embodiments, a light chain comprises at least a portion of a light chain constant region. The term "full-length light chain" refers to a polypeptide comprising a light chain variable region and a light chain constant region, with or without a leader sequence.

The term "hypervariable region" or "HVR" refers to each of the regions of the antibody variable domains which are hypervariable in sequence and/or form structurally defined loops ("hypervariable loops"). Generally, naturally occurring four-chain antibodies comprise six HVRs: three in the VH (H1, H2, and H3), and three in the VL (L1, L2, and L3). The HVR generally comprises amino acid residues from a hypervariable loop and/or from a "complementarity determining region" (CDR), whose sequence has the highest variability and/or which is involved in antigen recognition.

The term "antibody fragment" comprises a portion of a full-length antibody, generally at least the antigen-binding portion or variable region thereof. Examples of antibody fragments include diabodies, single-chain antibody molecules, conjugates such as immunotoxins, and multispecific antibodies comprising antibody fragments.

The term "humanized antibody" refers to an antibody in which the framework regions (FRs) and/or "complementarity determining regions" (CDRs) have been modified to comprise the CDR of an immunoglobulin of different specificity as compared to that of the parent immunoglobulin.

The term "epitope" denotes a protein determinant capable of specifically binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding of the antibody to the former but not the latter is lost in the presence of denaturing solvents.

The term "antibody-dependent cell-mediated cytotoxicity (ADCC)" denotes that killer cells such as NK cells directly kill target cells by recognizing the antibody Fc fragment coated on the target antigen through their expressed Fc receptors.

The term "fucose" in the present invention refers to fucose at Asn297 in the sugar chain of the antibody. The "fucose content" in the present invention refers to the sum of all fucose residues linked to Asn297, as measured by mass spectrometry and calculated as an average. The "relative amount of fucose" refers to the percentage of the structure containing fucose relative to all sugar structures, as identified by mass spectrometry in samples treated with IdeS.

The term "afucosylated antibody" is an IgG1 monoclonal antibody with a glycoform core structure dominated by afucosylated glycoforms and a fucose content not exceeding 5%.

The term "human effector cell" is a leukocyte which expresses one or more FcRs and performs effector functions. In a specific embodiment, the cell expresses at least FcyRIII and performs ADCC effector functions. Examples of human leukocytes which can mediate ADCC include peripheral blood mononuclear cells (PBMCs), natural killer (NK) cells, monocytes, macrophages, cytotoxic T cells, and neutrophils. Effector cells can be isolated from natural sources, such as from blood.

The term "combination therapy" refers to the administration of the anti-FGFR2IIIb antibody of the present invention in combination with chemotherapeutic drugs or immunosuppressants in a single or two separate formulations.

### Examples

### Example 1: Design and preparation of ADCC-enhanced anti-FGFR2b antibody

According to the amino acid sequences of heavy chain (SEQ ID NO: 12 or 14) and light chain (SEQ ID NO: 11) shown in Table 5 below, gene sequences were synthesized (General Biol, Chuzhou, Anhui), and cloned into the expression vector pcDNA^{™} 3.1 (Invitrogen, V79020), in order to separately prepare plasmids expressing heavy and light chains, which were both separately co-transfected into CHOK1 and Fut8KO CHOK1 cells according to Table 1 below. The transformed cells were cultured in 20% BM024H + 80% BM022H in a shaker at 120 rpm at 37°C and 6% CO₂ for one week, and the supernatant was collected (WuXi Biologics, Shanghai, China). The supernatant was purified by protein A affinity chromatography column (GE Healthcare). After purification, the purity of the antibodies was detected by the detection methods of SDS-PAGE (Thermo Fisher Scientific, NP0335BOX) and SEC-HPLC (Thermo, UltiMate3000), respectively. The purity of all antibodies was 95% or more.

**Table 1: Expression of antibody polypeptide chains**

| Sample batch No. | Host cell | Heavy chain | Light chain |
|---|---|---|---|
| FWB1914 | CHOK1 | SEQ ID NO: 12 | SEQ ID NO:11 |
| FWB19142 | CHOK1 | SEQ ID NO: 14 | SEQ ID NO:11 |
| FWB19143 | Fut8KO CHOK1 | SEQ ID NO:12 | SEQ ID NO:11 |
| FWB19144 | Fut8KO CHOK1 | SEQ ID NO:14 | SEQ ID NO:11 |

The antibodies were incubated with IdeS enzyme (Genovis, A0-FR1-020), and Fc/2 and F(ab')₂ fragments were obtained after digestion. The glycoforms were detected by using the ACQUITY UPLC I-Class/Acquity RDa system (Waters), and the relative content of a particular glycoform was analyzed based on the mass spectrometry peak area of the corresponding glycosyl group.

**Table 2: Analysis results of glycoforms**

| | FWB1914 | FWB19143 | FWB19142 | FWB19144 |
|---|---|---|---|---|
| Man5 | 5.04% | 4.28% | 1.67% | ND |
| G0-GN | ND | 3.69% | ND | ND |
| G0F-GN | 8.06% | ND | 1.4% | ND |
| G0 | 1.81% | 88.49% | ND | 87.98% |
| G0F | 63.98% | ND | 68.69% | ND |
| G1 | 13.15% | 3.54% | 13.91% | 12.02% |
| G1F | 7.97% | ND | 10.52% | ND |
| G0F+GN | ND | ND | 3.81% | ND |

| | | | | |
|---|---|---|---|---|
| ND: Not detectable | | | | |

As shown in Table 2, mass spectrometry analysis showed that the afucosylation in FWB19143 generated in host cells deficient in fucosylation, which had the same sequence as FWB1914, reached 95.77%; and similarly, the afucosylation in FWB19144 generated in host cells deficient in fucosylation, which had the same sequence as FWB 19142, reached 100%.

### Example 2: Identification of antigen binding activity of anti-FGFR2b antibody by ELISA

A 96-well ELISA plate (Coring, 3590) was first coated with 100 µL of 2 µg/mL rhFGFR2b-Fc (Sinobiological, 16485-H02H) overnight at 4°C, and incubated with 250 µL of blocking solution (PBST containing 3% BSA) for 2 hours at 37°C, and then washed with PBST for 3 times. The antibodies to be tested were subjected to a 5-fold series dilution from 15 µg/mL for a total of 8 concentration points (including a blank control), and the serially diluted antibodies were added to the ELISA well at 100 µL per well. The plate was incubated for 1 hour at 37°C, and then washed with PBST for 3 times. A secondary antibody mouse anti-human Fab-HRP conjugate (Genscript, A01855-200) was added. The plate was incubated for 1 hour at 37°C. Finally, the plate was washed with PBST for 3 times, and 100 µL of TMB (Biodragon, BF06007) was added for 15 minutes. The chromogenic reaction was terminated by using 2 N sulfuric acid (50 µL), and the absorbance value at 450 nm was detected on a plate reader (PerkinElmer, EnVision2104) to calculate the binding activity (EC50, nM) of each antibody to human FGFR2b.

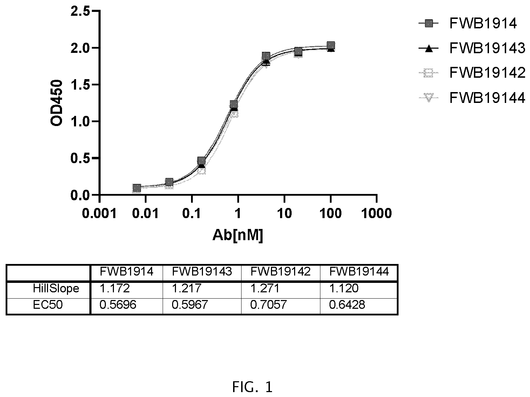

As shown in Figure 1, the EC50s of antibodies FWB1914, FWB19142, FWB19143, and FWB19144 ranged from 0.57 to 0.71 nM, demonstrating that the modification of the Fc region did not affect the antibody's binding activity to FGFR2b.

### Example 3: Detection of ADCC activity of antibodies by using Jurkat cells as effector cells

KATOIII (a gastric cancer cell line, Chinese Academy of Sciences Cell Bank, SCSP-573), SNU16 (a gastric cancer cell line, Zhongyuan, CRL-5974), or SUM52PE (a breast cancer cell line, BIOIVT, HUMANSUM-0003018) cells with high expression of FGFR2 were used as target cells, respectively. The density of the target cells was adjusted to 0.75 x 10⁶ cells/mL with phenol red-free RPMI1640 medium containing 10% FBS. The target cells were added to a white 96-well plate (Thermo, 136101) at 100 µL per well. The plate was cultured in a cell incubator overnight at 37°C and 5% CO₂. The following day, the culture supernatant was removed, 25 µL of serum-free, phenol red-free RPMI1640 was added to each well, and 25 µL of antibodies diluted at a 5-fold concentration gradient starting from a concentration of 1-5 µg/mL was added, with an equal volume of serum-free, phenol red-free RPMI1640 or negative samples added to the negative wells. The plate was incubated in a cell incubator at 37°C and 5% CO₂ for 60 minutes. The concentration of Jurkat (Suzhou Rhino Biotechnology, RA-CK01) effector cells was adjusted to 1 x 10⁶ cells/mL with serum-free, phenol red-free RPMI1640. 25 µL of cell suspension was added to each well. The plate was incubated in an incubator at 37°C and 5% CO₂ for 6 hours. The plate to be tested was allowed to return to room temperature, a firefly luciferase detection reagent (Vazyme, DD1204-01) was added, and the plate was incubated at room temperature in dark for 5 minutes, after which the luminescent value was detected.

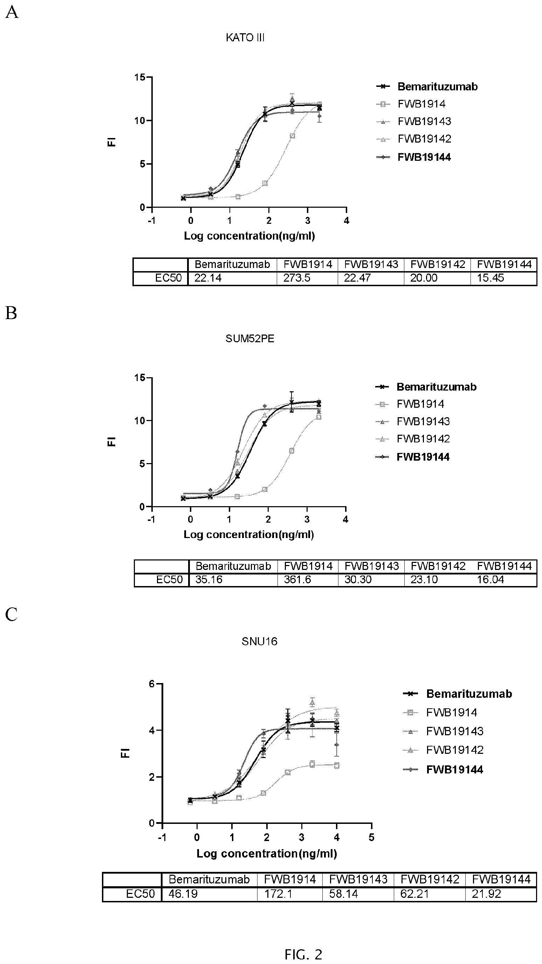

As shown in Figure 2, as detected by using Jurkat cells as effector cells, and KATOIII, SUM52PE, or SNU16 cells with high expression of FGFR2b as target cells, the anti-FGFR2b antibodies, which were afucosylated or had DLE mutations, exhibited stronger ADCC activity than the unmodified wild-type anti-FGFR2b antibody FWB1914. The fold enhancement (calculated based on EC50 (ng/mL)) in ADCC activity of each Fc-modified antibody over the wild-type anti-FGFR2b antibody FWB1914 was shown in Table 3.

**Table 3: Fold increase in ADCC potency of antibodies with modified Fc region over wild-type antibody**

| Target cell | FWB19142 | FWB19143 | FWB19144 |
|---|---|---|---|
| KATOIII | 14 | 12 | 18 |
| SUM52PE | 16 | 12 | 22 |
| SNU16 | 3 | 3 | 8 |

### Example 4: Detection of ADCC activity of antibodies by using human PBMCs as effector cells

KATOIII, SNU16, or SUM52PE cells were used as target cells, respectively. The density of the target cells was adjusted to 0.2 x 10⁶ cells/mL with phenol red-free RPMI1640 medium containing 10% FBS. The target cells were added to a 96-well plate (Corning, 3599) at 50 µL per well. The plate was cultured in a cell incubator overnight at 37°C and 5% CO₂. The following day, the culture supernatant was removed, 50 µL of phenol red-free RPMI1640 medium containing 1% FBS was added to each well, and 50 µL of antibodies diluted at a 5-fold concentration gradient starting from a concentration of 1-100 µg/mL was added, with an equal volume of serum-free, phenol red-free RPMI1640 or negative samples added to the negative wells. The plate was incubated in a cell incubator at 37°C and 5% CO₂ for 60 minutes. Human PBMC (Shanghai Saili Biotechnology, XFBHP025B) effector cells were thawed with phenol red-free RPMI1640 medium containing 10% FBS and IL-2 at a final concentration of 300 IU/mL, and cultured overnight. Then, the cells were centrifuged at 300 g for 10 minutes, resuspended with phenol red-free RPMI1640 containing 1% FBS, and counted. The concentration of the cells was adjusted to 5 x 10⁶ cells/mL. 50 µL of cell suspension was added to each well. The plate was incubated in a cell incubator at 37°C and 5% CO₂ for 6 hours. 50 µL of supernatant was sucked out from the well to be tested, 50 µL of LDH detection reagent (Promega, G1781) was added, and the plate was incubated at room temperature in dark for 30 minutes. After adding 50 µL of stop solution, the detection of OD492 was completed within 1 hour.

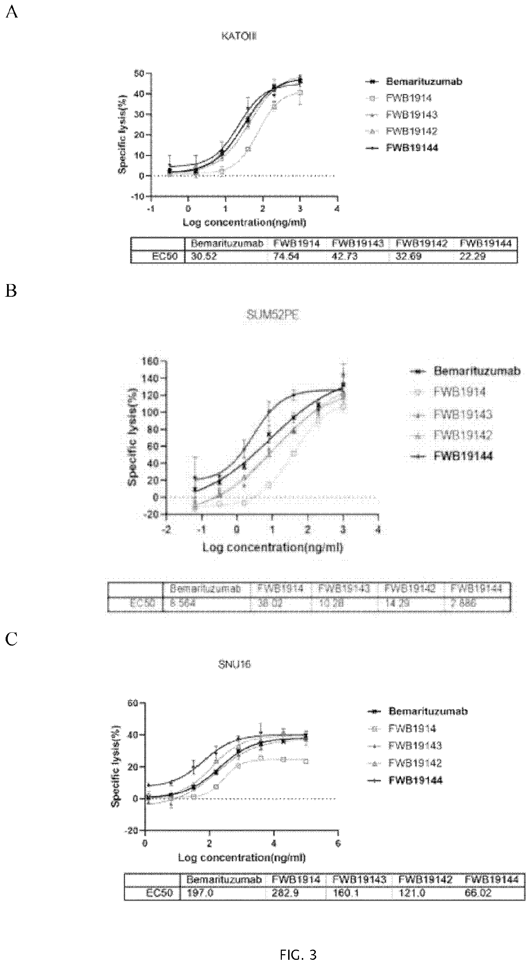

As shown in Figure 3, as detected by using human PBMC cells as effector cells, and KATOIII, SUM52PE, or SNU16 cells with high expression of FGFR2b as target cells, the anti-FGFR2b antibodies, which were afucosylated or had DLE mutations, exhibited stronger ADCC activity than the unmodified wild-type anti-FGFR2b antibody FWB1914. The fold enhancement (calculated based on EC50 (ng/mL)) in ADCC activity of each Fc-modified antibody over the wild-type anti-FGFR2b antibody FWB1914 was shown in Table 4.

**Table 4: Fold increase in ADCC potency of antibodies with modified Fc region over wild-type antibody**

| Target cell | FWB19142 | FWB19143 | FWB19144 |
|---|---|---|---|
| KATOIII | 2.3 | 1.7 | 3.4 |
| SUM52PE | 2.7 | 3.8 | 13.57 |
| SNU16 | 2.3 | 1.8 | 4.3 |

**Table 5: Sequences**

| |
|---|
| SEQ ID NO: 1, FGFR2b |
| MVSWGRFICLVVVTMATLSLARPSFSLVEDTTLEPEEPPTKYQISQPEVYVAAPGESLE VRCLLKDAAVISWTKDGVHLGPNNRTVLIGEYLQIKGATPRDSGLYACTASRTVDSET WYFMVNVTDAISSGDDEDDTDGAEDFVSENSNNKRAPYWTNTEKMEKRLHAVPAA NTVKFRCPAGGNPMPTMRWLKNGKEFKQEHRIGGYKVRNQHWSLIMESVVPSDKGN YTCVVENEYGSINHTYHLDVVERSPHRPILQAGLPANASTVVGGDVEFVCKVYSDAQ PHIQWIKHVEKNGSKYGPDGLPYLKVLKHSGINSSNAEVLALFNVTEADAGEYICKVS NYIGQANQSAWLTVLPKQQAPGREKEITASPDYLEIAIYCIGVFLIACMVVTVILCRMK NTTKKPDFSSQPAVHKLTKRIPLRRQVTVSAESSSSMNSNTPLVRITTRLSSTADTPML AGVSEYELPEDPKWEFPRDKLTLGKPLGEGCFGQVVMAEAVGIDKDKPKEAVTVAV KMLKDDATEKDLSDLVSEMEMMKMIGKHKNIINLLGACTQDGPLYVIVEYASKGNLR EYLRARRPPGMEYSYDINRVPEEQMTFKDLVSCTYQLARGMEYLASQKCIHRDLAAR NVLVTENNVMKIADFGLARDINNIDYYKKTTNGRLPVKWMAPEALFDRVYTHQSDV WSFGVLMWEIFTLGGSPYPGIPVEELFKLLKEGHRMDKPANCTNELYMMMRDCWHA VPSQRPTFKQLVEDLDRILTLTTNEEYLDLSQPLEQYSPSYPDTRSSCSSGDDSVFSPDP MPYEPCLPQYPHINGSVKT |
| |
| SEQ ID NO: 2, FGFR2c |
| |
| |
| SEQ ID NO: 3, VH (HVR is in bold) |
| QVQLVQSGAEVKKPGSSVKVSCKASGYIFTSYNVHWVRQAPGQGLEWIGSIYPDNGD SSYNNNYKGRATITADKSTSTAYMELSSLRSEDTAVYYCARGDFAYWGQGTLVTVSS |
| |
| SEQ ID NO: 4, VL (HVR is in bold) |
| DIQMTQSPSSLSASVGDRVTITC**KASNGVSNDIA**WYQQKPGKAPKLLIY**SASYRYS**GV PSRFSGSGSGTDFTFTISSLQPEDIATYYC**QQHSTTPYT**FGQGTKLEIK |
| |
| SEQ ID NO: 5, HVR-H1 |
| SYNVH |
| |
| SEQ ID NO: 6, HVR-H2 |
| SIYPDNGDSSYNNNYKG |
| |
| SEQ ID NO: 7, HVR-H3 |
| GDFAY |
| SEQ ID NO: 8, HVR-L1 |
| KASNGV SNDIA |
| |
| SEQ ID NO: 9, HVR-L2 |
| SASYRYS |
| |
| SEQ ID NO: 10, HVR-L3 |
| QQHSTTPYT |
| |
| SEQ ID NO: 11, light chain (variable domain is underlined) |
| DIQMTQSPSSLSASVGDRVTITCKASNGVSNDIAWYQQKPGKAPKLLIYSASYRYSGV PSRFSGSGSGTDFTFTISSLQPEDIATYYCQQHSTTPYTFGQGTKLEIKRTVAAPSVFIFP PSDEQLKSGTASVVCLLNNFYPREAKVQWKVDNALQSGNSQESVTEQDSKDSTYSLS STLTLSKADYEKHKVYACEVTHQGLSSPVTKSFNRGEC |
| |
| SEQ ID NO: 12, heavy chain (variable domain is underlined) |
| QVQLVQSGAEVKKPGSSVKVSCKASGYIFTSYNVHWVRQAPGQGLEWIGSIYPDNGD SSYNNNYKGRATITADKSTSTAYMELSSLRSEDTAVYYCARGDFAYWGQGTLVTVSS ASTKGPSVFPLAPSSKSTSGGTAALGCLVKDYFPEPVTVSWNSGALTSGVHTFPAVLQ |
| SSGLYSLSSVVTVPSSSLGTQTYICNVNHKPSNTKVDKRVEPKSCDKTHTCPPCPAPEL LGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGK |
| |
| SEQ ID NO: 13, Fc (DLE mutations) (point mutation is in bold) |
| PKSCDKTHTCPPCPAPELLGGPDVFLFPPKPKDTLMISRTPEVTCVVVDVSHEDPEVKF NWYVDGVEVHNAKTKPREEQYNSTYRVVSVLTVLHQDWLNGKEYKCKVSNKALPL PEEKTISKAKGQPREPQVYTLPPSREEMTKNQVSLTCLVKGFYPSDIAVEWESNGQPE NNYKTTPPVLDSDGSFFLYSKLTVDKSRWQQGNVFSCSVMHEALHNHYTQKSLSLSP GK |
| |
| SEQ ID NO: 14, heavy chain (DLE mutations) (variable domain is single-underlined, and Fc region is double-underlined) |
| QVQLVQSGAEVKKPGSSVKVSCKASGYIFTSYNVHWVRQAPGQGLEWIGSIYPDNGD SSYNNNYKGRATITADKSTSTAYMELSSLRSEDTAVYYCARGDFAYWGQGTLVTVSS ASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKRVEPKSCDKTHTCPPCPAPEL LGGPDVFLFPPKPKDTLMISRTPEVTCVVVDVSHEDPEVKFNWYVDGVEVHNAKTKP REEQYNSTYRVVSVLTVLHQDWLNGKEYKCKVSNKALPLPEEKTISKAKGQPREPQV YTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGK |

## Claims

1. An ADCC-enhanced anti-FGFR2 antibody, comprising a heavy chain variable region, a light chain variable region, and an Fc region, wherein the heavy chain variable region comprises:
(i) an HVR-H1 consisting of the amino acid sequence of SEQ ID NO: 5;
(ii) an HVR-H2 consisting of the amino acid sequence of SEQ ID NO: 6; and
(iii) an HVR-H3 consisting of the amino acid sequence of SEQ ID NO: 7,
the light chain variable region comprises:
(iv) an HVR-L1 consisting of the amino acid sequence of SEQ ID NO: 8;
(v) an HVR-L2 consisting of the amino acid sequence of SEQ ID NO: 9; and
(vi) an HVR-L3 consisting of the amino acid sequence of SEQ ID NO: 10,
and compared to a wild-type Fc region that is fucosylated, the Fc region exhibits enhanced binding to FcyRs.

2. The antibody according to claim 1, wherein the heavy chain variable region consists of the amino acid sequence of SEQ ID NO: 3, and/or the light chain variable region consists of the amino acid sequence of SEQ ID NO: 4.

3. The antibody according to claim 1 or 2, wherein the antibody comprises a κ light chain constant region and/or an IgG1 heavy chain constant region.

4. The antibody according to claim 1, wherein the heavy chain of the antibody consists of the amino acid sequence of SEQ ID NO: 12, and the light chain of the antibody consists of the amino acid sequence of SEQ ID NO: 11.

5. The antibody according to any one of claims 1 to 3, wherein the Fc region comprises a mutation that enhances binding to FcyRs, such as S239D/I332E or S239D/I332E/A330L mutations according to Kabat's EU index.

6. The antibody according to claim 1, wherein the heavy chain of the antibody consists of the amino acid sequence of SEQ ID NO: 14, and the light chain of the antibody consists of the amino acid sequence of SEQ ID NO: 11.

7. The antibody according to any one of claims 1 to 6, wherein the Fc region is afucosylated.

8. The antibody according to claim 7, wherein the antibody is generated in a host cell deficient in protein fucosylation.

9. The antibody according to claim 8, wherein the host cell is α-1,6-fucosyltransferase (FUT8)-deficient.

10. The antibody according to claim 7, wherein the antibody is generated under conditions where protein fucosylation is inhibited.

11. The antibody according to claim 10, wherein the conditions inhibit α-1,6-fucosyltransferase (FUT8).

12. The antibody according to claim 7, wherein the Fc region comprises a mutation that eliminates fucosylation at N297, such as an N297G or N297A mutation according to Kabat's EU index.

13. A composition, comprising the antibody according to any one of claims 1 to 12.

14. The composition according to claim 13, wherein at least 95% of the anti-FGFR2 antibodies in the composition are afucosylated.

15. A host cell, comprising a nucleic acid encoding the antibody according to any one of claims 1-6.

16. The host cell according to claim 15, wherein the host cell is deficient in protein fucosylation.

17. The host cell according to claim 16, wherein the host cell is FUT8-deficient.

18. A host cell, comprising a nucleic acid encoding the antibody according to claim 12.

19. A method for generating an ADCC-enhanced anti-FGFR2 antibody, comprising culturing the host cell according to claim 15 under conditions where protein fucosylation is inhibited.

20. A method for generating an ADCC-enhanced anti-FGFR2 antibody, comprising culturing the host cell according to claim 16 or 17 under conditions suitable for antibody production.

21. A method for generating an ADCC-enhanced anti-FGFR2 antibody, comprising culturing the host cell according to claim 18 under conditions suitable for antibody production.

22. A pharmaceutical composition, comprising the antibody according to any one of claims 1 to 12 or the composition according to claim 13 or 14, and a pharmaceutically acceptable carrier.

23. Use of the antibody according to any one of claims 1 to 12, the composition according to claim 13 or 14, or the pharmaceutical composition according to claim 22 in the preparation of a medicament for treating cancer in an individual.

24. The use according to claim 23, wherein the cancer overexpresses FGFR2.

25. The use according to claim 23 or 24, wherein the cancer is gastric cancer or breast cancer.

26. A method for treating cancer in an individual, comprising administering an effective amount of the antibody according to any one of claims 1-12, the composition according to claim 13 or 14, or the pharmaceutical composition according to claim 22 to the individual.

27. The method according to claim 26, wherein the cancer overexpresses FGFR2.

28. The method according to claim 26 or 27, wherein the cancer is gastric cancer or breast cancer.
